# EUROPEAN PATENT SPECIFICATION

(11) **EP 4 233 682 B1**
(45) Date of publication and mention of the grant of the patent: **07.10.2026**
(21) Application number: 23175494.6
(22) Date of filing: 01.11.2019
(51) Int. Cl.: A61B 1/00, A61B 1/018

(54) **ATTACHMENTS FOR ENDOSCOPES**
AUFSÄTZE FÜR ENDOSKOPE
ACCESSOIRES POUR ENDOSCOPES

(30) Priority: 02.11.2018 US 201862755024 P; 16.11.2018 US 201862768808 P; 15.04.2019 US 201962834192 P; 15.04.2019 US 201962834201 P
(43) Date of publication of application: 30.08.2023
(62) Document number(s) of the earlier application(s) in accordance with Art. 76 EPC: 19805746.5 / 3 817 638
(73) Proprietor: Boston Scientific Medical Device Limited, Galway (IE)
(72) Inventor: ANEJA, Harchetan Singh, Amritsar 143001 (IN); RAJARATHNAM, Boopathi, Salem 636005 (IN); UPADHYAY, Swami, Raipur 492013 (IN); RAWAT, Shalin Singh, Rishikesh 249201 (IN); NEELAMEGAM, Venkatesh, Tirupur 641602 (IN)
(74) Representative: Vossius & Partner Patentanwälte Rechtsanwälte mbB

(56) References cited:
- EP-A1- 2 505 119
- WO-A2-2005/011791

## Description

### Technical Field

The present disclosure relates generally to the field of medical instruments. More particularly, the present disclosure pertains to medical instruments for use with an endoscope, such as a biopsy cap and a biopsy cap housing with improved stability and stress distribution, for example, to securely attach to an endoscope biopsy port.

### Background

Conventional endoscope biopsy cap housings and biopsy caps can include a variety of deficiencies which may contribute - both individually and cumulatively - to component breakage, unnecessarily complicated or additional procedural steps and/or prolonged procedure times. For example, conventional biopsy cap housings tend to permit axial and rotational movement of the housing and/or cap during device exchange. In addition, exchange of larger diameter medical instruments (e.g., catheters, stent introducers, etc.) through the biopsy cap tends to exert a radially outward force which may cause the two center-split halves of conventional biopsy cap housings to partially or completely separate/disengage from each other. Adhesives applied to the center-split halves may minimize such separation but result in increased assembly time and cost. Locking or unlocking a guidewire to the hook(s) located on one side of a conventional biopsy cap housing tends to exert a radially outward force on one of the center-split halves, which may cause the center-split halves to move in opposite directions and partially or completely separate/disengage from each other. Excessive flexing due to lateral forces applied to one or both center-split halves, *e.g.,* during disengagement of the biopsy cap housing from the biopsy port, may concentrate stress on the locks which secure the biopsy cap housing to the endoscope port, resulting in a fracture of one or more of the locks. Any fracturing of components or separation between the center-split halves resulting from these forces may result in compromised stability between the biopsy cap housing and the endoscope biopsy port. In addition, the cumulative effects of these separation forces may decrease the operational longevity of the biopsy cap housing.

A variety of advantageous medical outcomes may therefore be realized by the biopsy cap and biopsy cap housing embodiments of the present disclosure. EP 2 505 119 A1, which discloses endoscope attachments according to the preambles of the independent claims, relates to medical instruments for use with an endoscope, such as a biopsy cap and a biopsy cap housing with improved stability and stress distribution to securely attach to an endoscope biopsy port.

### Brief Summary

The invention is defined by independent claims 1 and 11. Further embodiments of the invention are defined by the dependent claims. This disclosure provides design, material, manufacturing method, and use alternatives for medical instruments. The invention defined in claim 1 relates to an attachment for an endoscope which comprises: a housing; one or more locking members extending radially-inwardly from an inner surface of the housing, the locking members being designed to engage a biopsy port of an endoscope; one or more stabilizing members extending radially-inwardly from the inner surface of the housing; and a biopsy cap including one or more sealing members to seal against the biopsy port, wherein the biopsy cap is disposed within the housing.

Additionally, the one or more locking members may include a first locking member disposed on a first side of the inner surface of the housing and a second locking member disposed on a second side of the inner surface of the housing.

The one or more locking members may include a first locking member and wherein the first locking member includes a bent region.

The one or more locking members may include a first locking member and wherein the first locking member is substantially V-shaped.

The one or more locking members may be angled and include a first angled locking member and wherein the first angled locking member is substantially rigid
The one or more locking members may include a first locking member and wherein the first locking member is resiliently deflectable.

Optionally, the one or more stabilizing members include a first stabilizing member disposed on a first side of the inner surface of the housing and a second stabilizing member disposed on a second side of the inner surface of the housing.

The one or more stabilizing members include a first stabilizing member and wherein the first stabilizing member extends radially inward from the inner surface of the housing.

Optionally, the housing includes a skirt region.

Optionally, the locking apparatus includes one or more guidewire locks.

The invention as defined in independent claim 11 relates to an attachment for an endoscope. The attachment comprises: a housing designed to engage an endoscope and having a first end region and a second end region; a skirt region defined along a first end region of the housing and designed to engage around a portion of the endoscope; a locking member extending radially-inwardly from an inner surface of the housing between th efirst end region and the second end region of the housing to engage the biopsy port of an endoscope; a stabilizing member extending radially-inwardly from the inner surface of the housing between the first end region and the second end region of the housing and disposed adjacent to and circumferentially spaced apart from the locking member; and a biopsy cap including one or more sealing members to seal against the biopsy port, wherein the biopsy cap is disposed within the housing.

Optionally, the attachment further comprises a second locking member extending from the inner surface of the housing and disposed opposite the locking member.

Optionally, the locking member includes a bent region.

Optionally, the locking member is substantially V-shaped.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1 is a plan view of a portion of an including a biopsy port to a working channel of the endoscope.
FIG. 2 is a plan view of a biopsy cap housing coupled to an endoscope, in accordance with an embodiment of the present disclosure.
FIG. 3 is a side view of a portion of biopsy cap housing coupled to an endoscope, according to an embodiment of the present disclosure.
FIG. 4 is a perspective view of a biopsy cap housing, according to an embodiment of the present disclosure.
FIG. 5 is a side view of a portion of a biopsy cap housing coupled to an endoscope, according to an embodiment of the present disclosure.
FIG. 6 is a perspective view of a biopsy cap housing, according to an embodiment of the present disclosure.
FIGS. 7 and 8 depict a biopsy cap housing being coupled to an endoscope, according to an embodiment of the present disclosure.
FIG. 9 is a perspective view of a biopsy cap housing, according to an embodiment of the present disclosure.
FIGS. 10 and 11 depict a biopsy cap housing being coupled to an endoscope.

While the disclosure is amenable to various modifications and alternative forms, specifics thereof have been shown by way of example in the drawings and will be described in detail. It should be understood, however, that the intention is not to limit the invention to the particular embodiments described. On the contrary, the invention is defined in the appended claims.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range *(e.g.* 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

In various embodiments, features and advantages of providing sealable access to a working channel, *e.g.,* of an endoscope, may be realized in combination with a biopsy cap and biopsy cap housing. Such sealable access to a working channel, which may be reinforced, may be implemented with features throughout the disclosures of United States Patent Application Serial No. 16/100,960, filed August 10, 2018 and titled "Biopsy Cap For Use With Endoscope," United States Patent Application having an Attorney Docket No. 8150.0657, filed on even date herewith and titled "Devices, Systems, And Methods For A Biopsy Cap And Housing," United States Patent Application having an Attorney Docket No. 8150.0553, filed on even date herewith and titled "Biopsy Cap And Biopsy Cap Housing," United States Patent Application having an Attorney Docket No. 8150.0656 filed on even date herewith and titled, "Devices, Systems, And Methods For Providing Sealable Access To A Working Channel," United States Patent Application having an Attorney Docket No. 8150.0610, filed on even date herewith and titled "Internal Seal for Biopsy Cap," United States Patent Application having an Attorney Docket No. 8150.0555, filed on even date herewith and titled "Devices, Systems, and Methods for Providing Sealable Access to a Working Channel".

During endoscopic procedures, a medical instrument such as a guidewire, catheter, endoscopic instrument, or the like may be inserted through a working channel of the endoscope. A port *(e.g.,* a "biopsy port") along the endoscope may provide access to the working channel. During use, it may be desirable to couple a biopsy cap to the biopsy port. The biopsy cap may have one more seal or sealing members. The seals may be designed to seal against the biopsy port and/or against instrument(s) that may be extended through the biopsy cap into the working channel. During some interventions, it may be desirable to secure the position of a medical instrument (e.g., a guidewire) relative to the endoscope. In order to secure the medical instrument, a locking mechanism may be secured to the endoscope and/or the biopsy cap. Disclosed herein are endoscope attachments or biopsy cap assemblies that may include a number of features including a biopsy cap housing, biopsy cap, a locking mechanism, as well as other features.

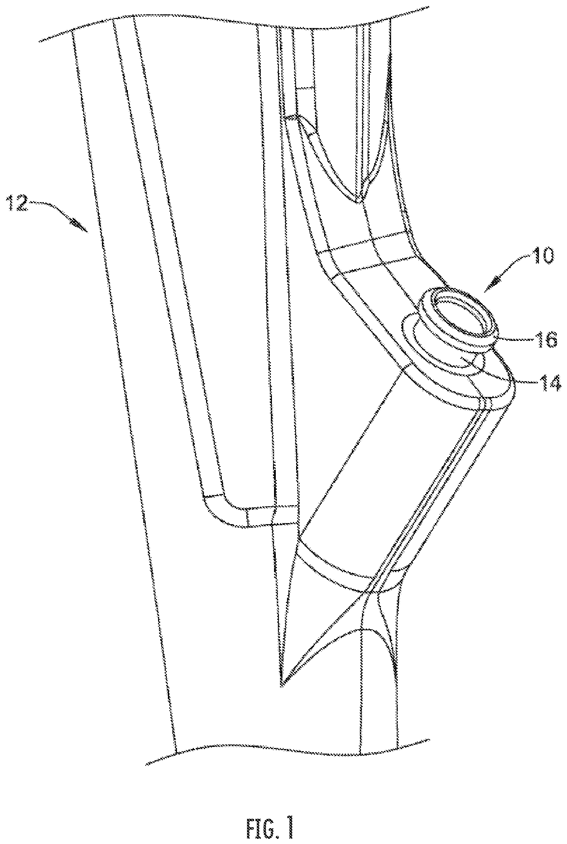

FIG. 1 illustrates a portion of a biopsy port 10 of an example endoscope 12. The biopsy port 10 may include a stem or neck region 14 and an end or flanged region 16. The biopsy port 10 serves as an access point to a channel *(e.g.,* a working channel) of the endoscope 12. The biopsy port 10, in general, may be designed to receive a biopsy cap.

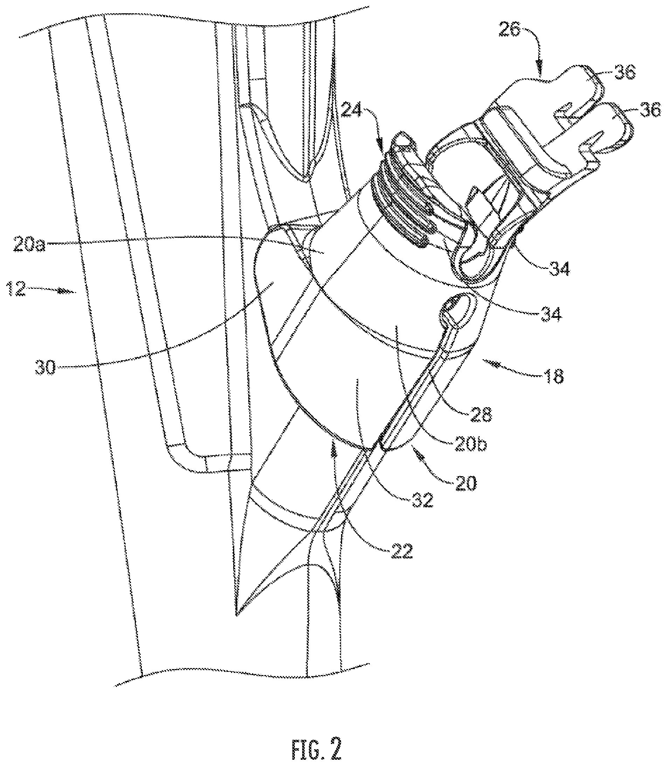

FIGS. 2-3 illustrates an example endoscope attachment 18 including a housing 20 coupled to the biopsy port 10. In general, the endoscope attachment 18 is designed to be coupled to *(e.g.,* detachably coupled, attached, secured to, etc.) the biopsy port of an endoscope *(e.g.,* the biopsy port 10 of the endoscope 12). In addition, the endoscope attachment 18 includes a number of features including the ability to form a seal with a biopsy cap at the biopsy port 10, allow for a medical instrument (e.g., a guidewire, catheter, endoscopic device, and/or the like) to pass therethrough and into the working channel of the endoscope 12, allow for the medical instrument to be secured relative to the endoscope 12, etc., and may be configured to securely receive a biopsy cap.

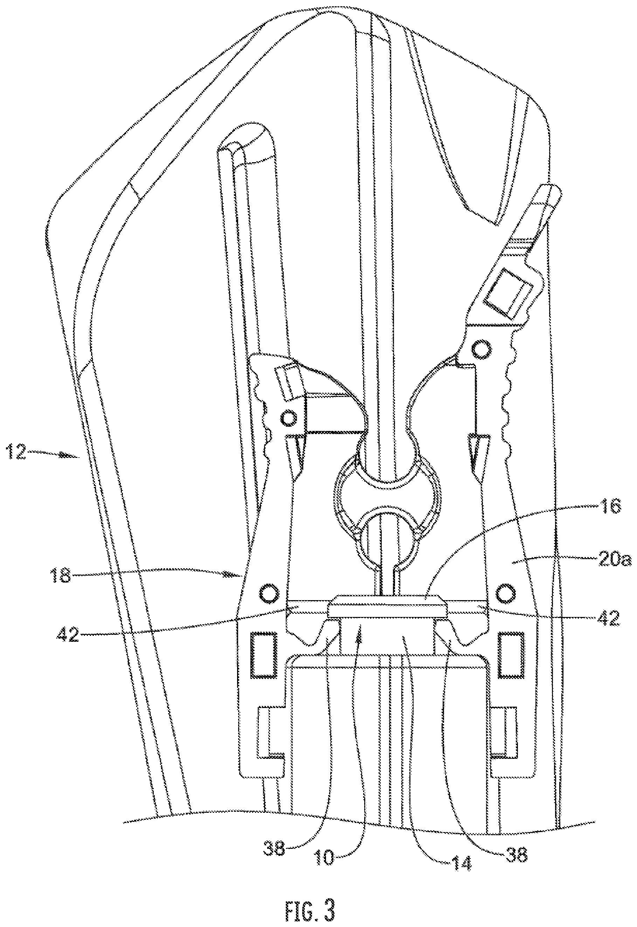

The endoscope attachment 18 may include a housing 20 having a skirt region 22, a grip region 24, and a locking region 26. In some instances, the housing 20 may be a single piece. In other instances, the housing 20 may be formed from 2 or more pieces, for example, first housing portion 20a and second housing portion 20b, which are secured together. In FIG. 3, for example, the second housing portion 20b is removed and only the first housing portion 20a is shown. Forming the housing 20 from separate pieces may allow for the housing 20 to be relatively easily molded (e.g., compared to a singular piece) or otherwise formed into a complex shape. When formed from separate pieces, the housing portions 20a/20b may be secured together in a suitable manner such as by pinning (e.g., snap locks that include pins on one portion and holes or openings to receive the pins on the other portion), thermal bonding, adhesive bonding, and/or the like. In various embodiments, the housing 20 may be substantially rigid. Pinning may result in secure attachment of the portions 20a, 20b and also may enhance the ability of the two portions 20a/20b of the housing to "pivot" relative to one another. When doing so, the housing 20 can open or widen (e.g., adjacent to a lower portion of the endoscope attachment 18 such as adjacent to the skirt region 22) in order to attached/detach the endoscope attachment 18 to the biopsy port 10. A slot or opening 28 may be formed along a portion of the housing 20. The slot(s) may enhance the flexibility and/or pivoting ability of the housing 20 and may make it easier to secure the endoscope attachment 18 to the biopsy port 10.

The skirt region 22 may generally be designed to follow the shape and/or contour of the endoscope 12. More particularly, the skirt region 22 may be shaped so as to conform to the shape of the handle region of the endoscope 12 adjacent to the biopsy port 10. This may increase the stability of the endoscope attachment 18 and assist with securing hold the position of the endoscope attachment 18 relative to the endoscope 12. In at least some instances, the shape of the skirt region 22 may be described as being asymmetrical. For example, the skirt region 22 may include a first portion 30 *(e.g.,* which may or may not correspond to the first housing portion 20a) and a second portion 32 *(e.g.,* which may or may not correspond to the second housing portion 20b).

The grip region 24 may include one or more gripping members 34. The gripping members 34 may take the form of finger or pinch grips that allow a user to grasp the endoscope attachment 18. In some instances, pinching together the gripping members 34 may widen the endoscope attachment 18 *(e.g.,* adjacent to the skirt region 22) and allow for the endoscope attachment 18 to be more attached to/detached from the biopsy port 10. The slot 28 may help to facilitate the flexing/bending of the endoscope attachment 18 when the gripping members 34 are pinched.

The locking region 26 may include one or more locking apparatuses 36. The locking apparatuses 36 may vary in form. In some instances, the locking apparatuses 36 may take the form of hooks designed to engage and hold a medical instrument (e.g., a guidewire, catheter, endoscopic device, and/or the like). When doing so, the medical instrument can be held in place relative to the endoscope attachment 18 (and/or the endoscope 12).

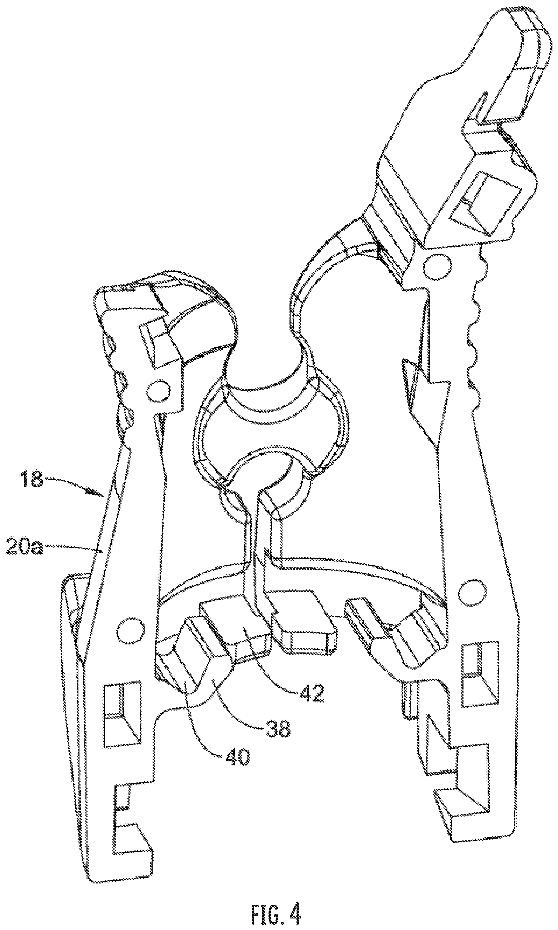

As shown in FIGS. 3 and 4, housing 20 of the endoscope attachment 18 may include one or more angled locking members 38. In general, the angled locking members 38 take the form of projections that extend radially inward and in a direction toward an upper chamber from the inner surface of the housing 20. The angled locking members 38 may include a bend, jogged, or bent region 40. In at least some instances, the angled locking members 38 may be described as being "V-shaped". However, other shapes are contemplated. The angled locking members 38 may be resiliently flexible such that the angled locking members 38 can bend or deflect in order to fit over and secure to the flanged region 16 of the biopsy port 10. The number of angled locking members 38 and/or the arrangement of the angled locking members 38 may vary. For example, the housing 20 may include two, three, four, five, six, seven, eight, or more angled locking members 38. In some instances, each center-split halves 20a, 20b includes a pair of angled locking members 38 that are arranged across from or opposite one another. When the housing portions d20a, 20b are brought together, one of the angled locking members 38 from each of the opposing housing portions 20a, 20b may be disposed adjacent to one another. Other arrangements are contemplated.

The endoscope attachment 18 may include one more stabilizing member 42. In general, the stabilizing members 42 may take the form of take the form of projections that extend radially inward from the inner surface of the housing 20. The stabilizing members 42 may help to stabilize the position (e.g., laterally and/or axially) on the biopsy port 10. The number of stabilizing members 42 and/or the arrangement of the stabilizing members 42 may vary. For example, the endoscope attachment 18 may include two, three, four, five, six, seven, eight, or more stabilizing members 42. In some instances, each housing portion 20a/20b includes a pair of stabilizing members 42 that are disposed adjacent to one another. When the housing portions 20a/20b are brought together, the pairs of stabilizing members 42 from each of the opposing housing portions 20a/20b may be disposed opposite to one another. Other arrangements are contemplated.

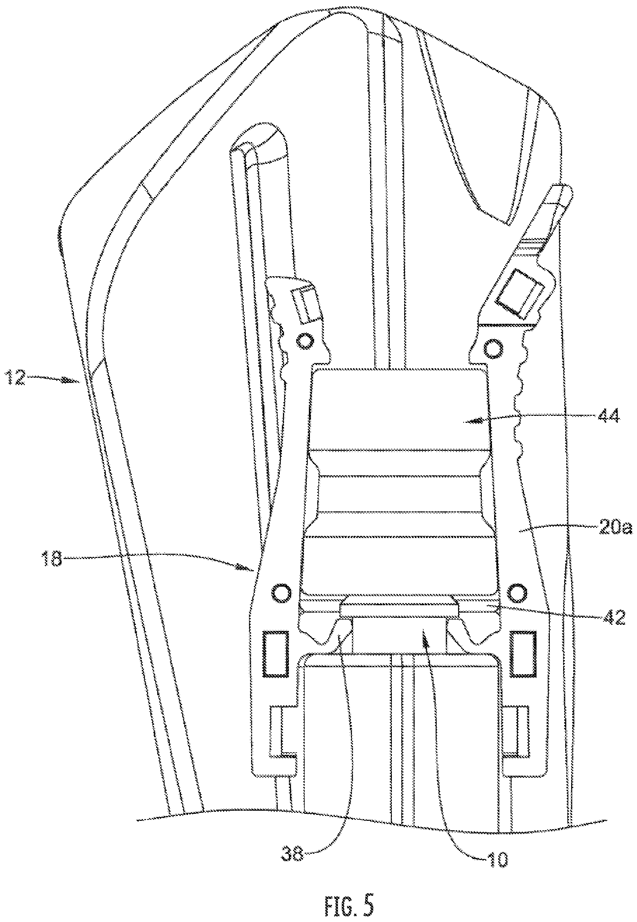

A biopsy cap 44 may be disposed within the housing 20 of the endoscope attachment 18 as shown in FIG. 5. It is noted that for clarity purposes, the biopsy cap 44 is not shown in FIGS. 3-4. The biopsy cap 44 may vary in form. In at least some instances, the biopsy cap 44 may include one or more seals or sealing members (not depicted in FIG. 5). The seal(s) may be designed to seal against the biopsy port 10 and thereby prevent fluid from leaking from the biopsy port 10. In addition, the seal(s) may be designed to seal against a medical instrument (e.g., a guidewire, catheter, endoscopic device, and/or the like) passing through the endoscope attachment 18 and/or the biopsy cap 44. In at least some instances, the biopsy cap 44 may be disposed adjacent to and/or otherwise seated on the stabilizing members 42.

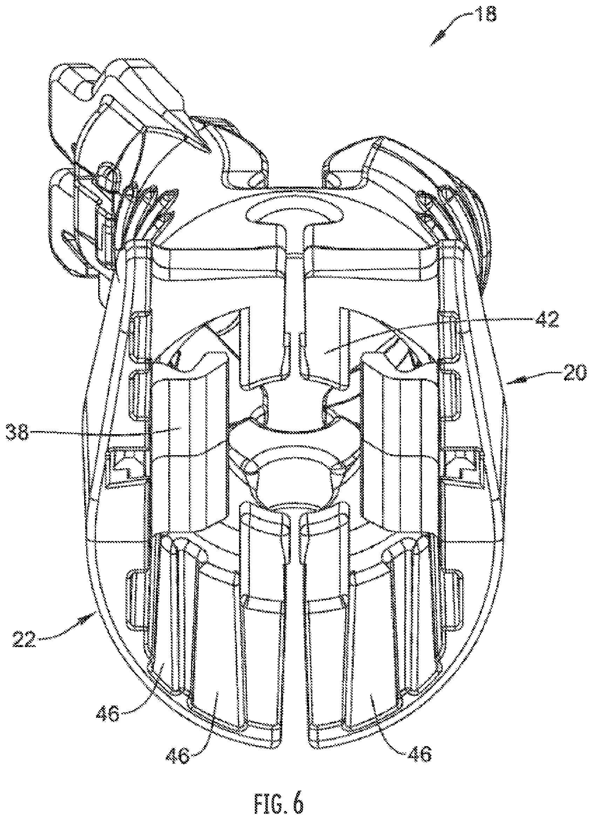

**In** an embodiment, a skirt region 22 may have one or more gripping members or ribs 46 disposed along an inner surface (e.g., an inner surface of the housing 20 at or along the skirt region 22) as shown in FIG. 6. The ribs 46 may help to form or otherwise define a surface along the interior of the endoscope attachment 18 that allows the endoscope attachment 18 to "grip" onto and/or otherwise frictionally engage the endoscope 12 and, thus, help to secure the endoscope attachment 18 to the endoscope 12.

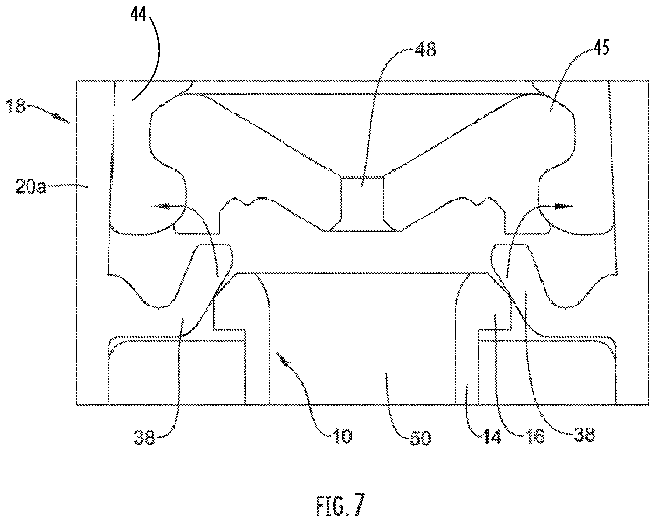

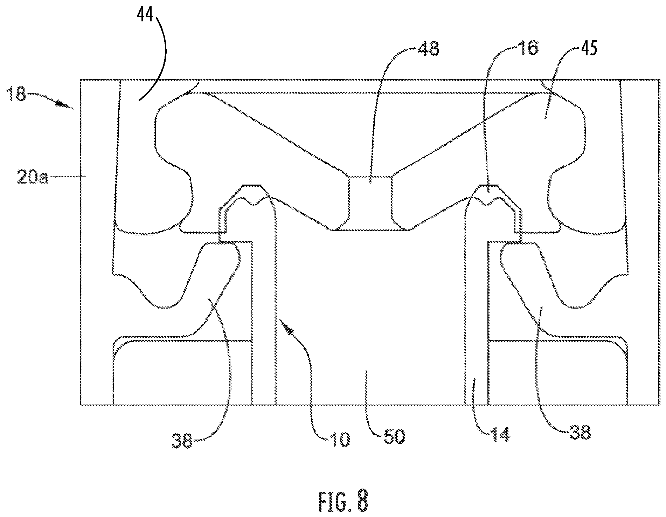

FIGS. 7 and 8 depict an embodiment of an endoscope attachment 18 with housing 20 being attached to a biopsy port 10. For example, the endoscope attachment 18 may be disposed adjacent to the biopsy port 10. When doing so, the angled locking members 38 may be brought adjacent to the flanged region 16 of the biopsy port as shown in FIG. 7. A user may apply a force *(e.g.,* a pinching force) along a grip region *(e.g.,* the grip region 24 of FIG. 2) of the attachment 18 to outwardly flex or otherwise pivot in order to open or widen the housing 20 (e.g., adjacent to the skirt region 22). The user may also apply a downward force onto the endoscope attachment 18 to bring the endoscope attachment into engagement with and secure the endoscope attachment 18 to the biopsy port 10. When doing so, the angled locking members 38 may resiliently deflect so that the angled locking members 38 are seated underneath the flanged region as shown in FIG. 8. When secured, the stabilizing members 42 may engage the neck region 14 and/or the flanged region 16 of the biopsy port 10. Securing the endoscope attachment 18 to the biopsy port 10 may also bring an aperture 48 of seal member 44 or a base 45 into engagement with the biopsy port 10 so that the seal member 48 may press against or otherwise seal a channel 50 of the endoscope 12 *(e.g., a* channel 50 accessible via the biopsy port 10). When the endoscope attachment 18 is secured to the biopsy port 10, the endoscope attachment 18 may provide haptic feedback such as a "snap" or "click" sound and/or sensation, and/or the like. A user may remove the endoscope attachment 18 by simply pinching the grip region 24 in order to flex/pivot/widen the housing 20 (as well as widen/open the angled locking members 38) and pulling the endoscope attachment 18 from the biopsy port 10.

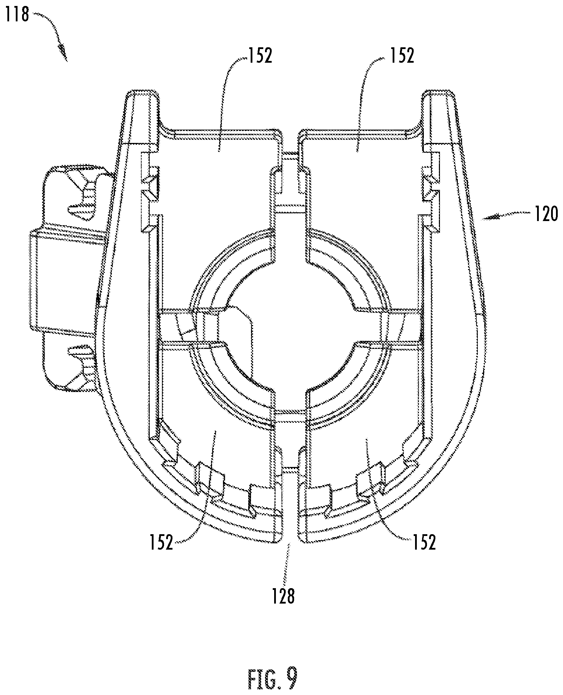

FIG. 9 illustrates an embodiment of an endoscope attachment 118 that may be similar in form and function to other endoscope attachments disclosed herein. The endoscope attachment 118 may include a housing 120. The housing 120 may be generally more flexible than the housing 20. A slot 128 may be formed in the housing to further enhance the flexibility of the housing. One or more locking members 152 may extend from the housing 120. In some instances, the locking members 152 may take the form of substantially rigid projections that extend radially inward from the housing 120. In at least some instances, the locking members 152 may be arranged to form a generally circular *(e.g.,* and/or a broken circle) lock designed to extend around a biopsy port *(e.g.,* such as a biopsy port 10.

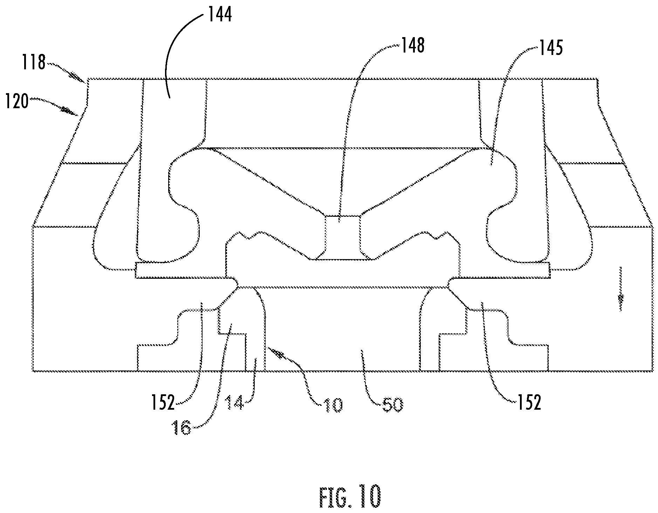

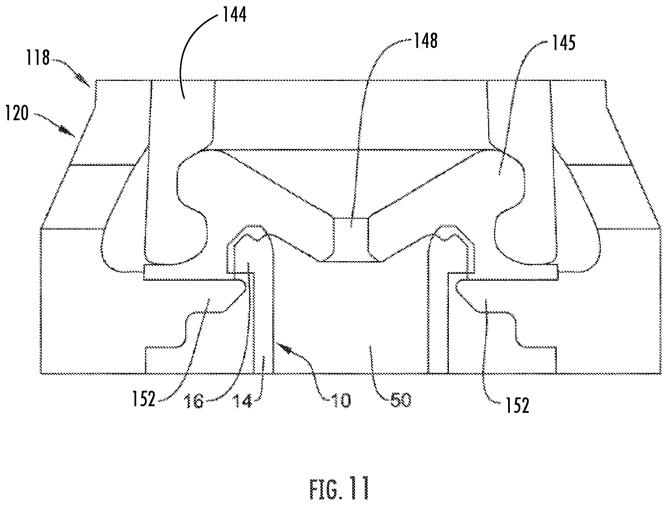

FIGS. 10 and 11 depict the endoscope attachment 118 being attached to a biopsy port 10. For example, the endoscope attachment 118 may be disposed adjacent to the biopsy port 10. When doing so, the locking members 152 may be brought adjacent to the flanged region 16 of the biopsy port as shown in FIG. 10. A user may apply a downward force onto the endoscope attachment 118 to secure the endoscope attachment 118 to the biopsy port 10. When doing so, the housing 120 may resiliently deflect so that the locking members 152 may be seated underneath the flanged region as shown in FIG. 11. Securing the endoscope attachment 118 to the biopsy port 10 may also bring an aperture 148 of the seal member 144 or a base 145 into engagement with the biopsy port 10 so that the base 145 may press against or otherwise seal a channel 50 of the endoscope 12 *(e.g.,* a channel 50 accessible via the biopsy port 10). When the endoscope attachment 118 is secured to the biopsy port 10, the endoscope attachment 118 may provide haptic feedback such as a "snap" or "click" sound.

The materials that can be used for the various components of the endoscope attachment 18 (and/or other endoscope attachments disclosed herein) may include those commonly associated with medical instruments. For example, the endoscope attachment 18 may be made from a metal, metal alloy, polymer (some examples of which are disclosed below), a metal-polymer composite, ceramics, combinations thereof, and the like, or other suitable material. Some examples of suitable polymers may include acrylonitrile butadiene styrene, acrylonitrile butadiene styrene and polycarbonate, polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), Marlex high-density polyethylene, Marlex low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-6, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like.

## Claims

1. An attachment (118) for an endoscope (12), the attachment (118) comprising:
a housing (120);
one or more locking members (152) extending radially-inwardly from an inner surface of the housing (120), the locking members (152) being designed to engage a biopsy port (10) of an endoscope (12); and
one or more stabilizing members (42) extending radially-inwardly from the inner surface of the housing (120) and circumferentially spaced apart from the locking members (152) to stabilize a lateral position of the attachment (118) with respect to the biopsy port (10); **characterised in that** the attachment further comprises:
a biopsy cap (44) including one or more sealing members to seal against the biopsy port (10), wherein the biopsy cap (44) is disposed within the housing (120).

2. The attachment (118) of claim 1, wherein the one or more locking members (152) include a first locking member (152) disposed on a first side of the inner surface of the housing (120) and a second locking member (152) disposed on a second side of the inner surface of the housing (120).

3. The attachment (118) of any one of claims 1-2, wherein the one or more locking members (152) include a first locking member (152), and wherein the first locking member includes a bent region (40).

4. The attachment (118) of any one of claims 1-3, wherein the one or more locking members (152) include a first locking member (152) and wherein the first locking member (152) is substantially V-shaped.

5. The attachment (118) of any one of claims 1-4, wherein the one or more locking members (152) are angled and include a first angled locking member (152) and wherein the first angled locking member (152) is substantially rigid.

6. The attachment (118) of any one of claims 1-4, wherein the one or more locking members (152) include a first locking member (152) and wherein the first locking member (152) is resiliently deflectable.

7. The attachment (118) of any one of claims 1-6, wherein the one or more stabilizing members (42) include a first stabilizing member (42) disposed on a first side of the inner surface of the housing (120) and a second stabilizing member (42) disposed on a second side of the inner surface of the housing (120).

8. The attachment (118) of any one of claims 1-6, wherein the one or more stabilizing members (42) include a first stabilizing member (42) and wherein the first stabilizing member (42) extends radially inward from the inner surface of the housing (120).

9. The attachment (118) of any one of claims 1-8, wherein the housing (120) includes a skirt region (22).

10. The attachment (118) of any one of claims 1-9, further comprising a locking apparatus (36) including one or more guidewire locks.

11. An attachment (118) for an endoscope (12), the attachment (118) comprising:
a housing (120) designed to engage an endoscope (12) and having a first end region and a second end region;
a skirt region (22) defined along the first end region of the housing (120) and designed to engage around a portion of the endoscope (118);
a locking member (152) extending radially-inwardly from an inner surface of the housing (120) between the first end region and the second end region of the housing (120) to engage the biopsy port (10) of an endoscope (12);
a stabilizing member (42) extending radially-inwardly from the inner surface of the housing (120) between the first end region and the second end region of the housing (120) and disposed adjacent to and circumferentially spaced apart from the locking member (152); **characterised in that** the attachment further comprises:
a biopsy cap (44) including one or more sealing members to seal against the biopsy port (10), wherein the biopsy cap (44) is disposed within the housing (120).

12. The attachment (118) of claim 11, further comprising a second locking member (152) extending from the inner surface of the housing (120) and disposed opposite the locking member (152).

13. The attachment (118) of any one of claims 11-12, wherein the locking member (152) includes a bent region (40).

14. The attachment (118) of any one of claims 11-13, wherein the locking member (152) is substantially V-shaped.

## Patentansprüche

1. Aufsatz (118) für ein Endoskop (12), wobei der Aufsatz (118) aufweist:
ein Gehäuse (120);
ein oder mehrere Verriegelungsteile (152), die sich von einer Innenfläche des Gehäuses (120) radial nach innen erstrecken, wobei die Verriegelungsteile (152) so gestaltet sind, dass sie einen Eingriff mit einem Biopsie-Port (10) eines Endoskops (12) herstellen; und
ein oder mehrere Stabilisierungsteile (42), die sich von der Innenfläche des Gehäuses (120) radial nach innen erstrecken und von den Verriegelungsteilen (152) über den Umfang beabstandet sind, um eine Lateralposition des Aufsatzes (118) im Hinblick auf den Biopsie-Port (10) zu stabilisieren; **dadurch gekennzeichnet, dass** der Aufsatz ferner aufweist:
eine Biopsiekappe (44) mit einem oder mehreren Dichtungsteilen, um am Biopsie-Port (10) abzudichten, wobei die Biopsiekappe (44) im Gehäuse (120) angeordnet ist.

2. Aufsatz (118) nach Anspruch 1, wobei das eine oder die mehreren Verriegelungsteile (152) aufweisen: ein erstes Verriegelungsteil (152), das auf einer ersten Seite der Innenfläche des Gehäuses (120) angeordnet ist, und ein zweites Verriegelungsteil (152), das auf einer zweiten Seite der Innenfläche des Gehäuses (120) angeordnet ist.

3. Aufsatz (118) nach einem der Ansprüche 1 bis 2, wobei das eine oder die mehreren Verriegelungsteile (152) ein erstes Verriegelungsteil (152) aufweisen und wobei das erste Verriegelungsteil einen Biegebereich (40) aufweist.

4. Aufsatz (118) nach einem der Ansprüche 1 bis 3, wobei das eine oder die mehreren Verriegelungsteile (152) ein erstes Verriegelungsteil (152) aufweisen und wobei das erste Verriegelungsteil (152) im Wesentlichen V-förmig ist.

5. Aufsatz (118) nach einem der Ansprüche 1 bis 4, wobei das eine oder die mehreren Verriegelungsteile (152) abgewinkelt sind und ein erstes abgewinkeltes Verriegelungsteil (152) aufweisen und wobei das erste abgewinkelte Verriegelungsteil (152) im Wesentlichen steif ist.

6. Aufsatz (118) nach einem der Ansprüche 1 bis 4, wobei das eine oder die mehreren Verriegelungsteile (152) ein erstes Verriegelungsteil (152) aufweisen und wobei das erste Verriegelungsteil (152) elastisch ablenkbar ist.

7. Aufsatz (118) nach einem der Ansprüche 1 bis 6, wobei das eine oder die mehreren Stabilisierungsteile (42) ein erstes Stabilisierungsteil (42), das auf einer ersten Seite der Innenfläche des Gehäuses (120) angeordnet ist, und ein zweites Stabilisierungsteil (42) aufweisen, das auf einer zweiten Seite der Innenfläche des Gehäuses (120) angeordnet ist.

8. Aufsatz (118) nach einem der Ansprüche 1 bis 6, wobei das eine oder die mehreren Stabilisierungsteile (42) ein erstes Stabilisierungsteil (42) aufweisen und wobei sich das erste Stabilisierungsteil (42) von der Innenfläche des Gehäuses (120) radial nach innen erstreckt.

9. Aufsatz (118) nach einem der Ansprüche 1 bis 8, wobei das Gehäuse (120) einen Schürzenbereich (22) aufweist.

10. Aufsatz (118) nach einem der Ansprüche 1 bis 9, ferner mit einer Verriegelungsvorrichtung (36), die eine oder mehrere Führungsdrahtverriegelungen aufweist.

11. Aufsatz (118) für ein Endoskop (12), wobei der Aufsatz (118) aufweist:
ein Gehäuse (120), das so gestaltet ist, dass es einen Eingriff mit einem Endoskop (12) herstellt, und einen ersten Endbereich und einen zweiten Endbereich hat;
einen Schürzenbereich (22), der entlang des ersten Endbereichs des Gehäuses (120) definiert und so gestaltet ist, dass er einen Eingriff um einen Abschnitt des Endoskops (118) herstellt;
ein Verriegelungsteil (152), das sich von einer Innenfläche des Gehäuses (120) zwischen dem ersten Endbereich und dem zweiten Endbereich des Gehäuses (120) radial nach innen erstreckt, um einen Eingriff mit dem Biopsie-Port (10) eines Endoskops (12) herzustellen;
ein Stabilisierungsteil (42), das sich von der Innenfläche des Gehäuses (120) zwischen dem ersten Endbereich und dem zweiten Endbereich des Gehäuses (120) radial nach innen erstreckt und benachbart zum Verriegelungsteil (152) und davon über den Umfang beabstandet angeordnet ist; **dadurch gekennzeichnet, dass** der Aufsatz ferner aufweist:
eine Biopsiekappe (44) mit einem oder mehreren Dichtungsteilen, um am Biopsie-Port (10) abzudichten, wobei die Biopsiekappe (44) im Gehäuse (120) angeordnet ist.

12. Aufsatz (118) nach Anspruch 11, ferner mit einem zweiten Verriegelungsteil (152), das sich von der Innenfläche des Gehäuses (120) erstreckt und entgegengesetzt zum Verriegelungsteil (152) angeordnet ist.

13. Aufsatz (118) nach einem der Ansprüche 11 bis 12, wobei das Verriegelungsteil (152) einen Biegebereich (40) aufweist.

14. Aufsatz (118) nach einem der Ansprüche 11 bis 13, wobei das Verriegelungsteil (152) im Wesentlichen V-förmig ist.

## Revendications

1. Accessoire (118) pour un endoscope (12), l'accessoire (118) comprenant:
un boîtier (120);
un ou plusieurs éléments de verrouillage (152) s'étendant radialement vers l'intérieur depuis une surface interne du boîtier (120), les éléments de verrouillage (152) étant conçus pour coopérer avec un port de biopsie (10) d'un endoscope (12); et
un ou plusieurs éléments de stabilisation (42) s'étendant radialement vers l'intérieur depuis la surface interne du boîtier (120) et espacés circonférentiellement des éléments de verrouillage (152) pour stabiliser une position latérale de l'accessoire (118) par rapport au port de biopsie (10); **caractérisé en ce que** l'accessoire comprend en outre:
un capuchon de biopsie (44) incluant un ou plusieurs éléments d'étanchéité pour assurer l'étanchéité contre le port de biopsie (10), où le capuchon de biopsie (44) est disposé à l'intérieur du boîtier (120).

2. Accessoire (118) selon la revendication 1, où les un ou plusieurs éléments de verrouillage (152) incluent un premier élément de verrouillage (152) disposé sur un premier côté de la surface interne du boîtier (120) et un second élément de verrouillage (152) disposé sur un second côté de la surface interne du boîtier (120).

3. Accessoire (118) selon l'une quelconque des revendications 1 et 2, où les un ou plusieurs éléments de verrouillage (152) incluent un premier élément de verrouillage (152), et où le premier élément de verrouillage inclut une région courbée (40).

4. Accessoire (118) selon l'une quelconque des revendications 1 à 3, où les un ou plusieurs éléments de verrouillage (152) incluent un premier élément de verrouillage (152) et où le premier élément de verrouillage (152) est sensiblement en forme de V.

5. Accessoire (118) selon l'une quelconque des revendications 1 à 4, où les un ou plusieurs éléments de verrouillage (152) sont inclinés et incluent un premier élément de verrouillage incliné (152) et où le premier élément de verrouillage incliné (152) est sensiblement rigide.

6. Accessoire (118) selon l'une quelconque des revendications 1 à 4, où les un ou plusieurs éléments de verrouillage (152) incluent un premier élément de verrouillage (152) et où le premier élément de verrouillage (152) peut être dévié de manière élastique.

7. Accessoire (118) selon l'une quelconque des revendications 1 à 6, où les un ou plusieurs éléments de stabilisation (42) incluent un premier élément de stabilisation (42) disposé sur un premier côté de la surface interne du boîtier (120) et un second élément de stabilisation (42) disposé sur un second côté de la surface interne du boîtier (120).

8. Accessoire (118) selon l'une quelconque des revendications 1 à 6, où les un ou plusieurs éléments de stabilisation (42) incluent un premier élément de stabilisation (42) et où le premier élément de stabilisation (42) s'étend radialement vers l'intérieur depuis la surface interne du boîtier (120).

9. Accessoire (118) selon l'une quelconque des revendications 1 à 8, où le boîtier (120) inclut une région formant jupe (22).

10. Accessoire (118) selon l'une quelconque des revendications 1 à 9, comprenant en outre un appareil de verrouillage (36) incluant un ou plusieurs verrous de fil de guidage.

11. Accessoire (118) pour un endoscope (12), l'accessoire (118) comprenant:
un boîtier (120) conçu pour coopérer avec un endoscope (12) et ayant une première région d'extrémité et une seconde région d'extrémité;
une région formant jupe (22) définie le long de la première région d'extrémité du boîtier (120) et conçue pour coopérer autour d'une partie de l'endoscope (118);
un élément de verrouillage (152) s'étendant radialement vers l'intérieur depuis une surface interne du boîtier (120) entre la première région d'extrémité et la seconde région d'extrémité du boîtier (120) pour coopérer avec le port de biopsie (10) d'un endoscope (12);
un élément de stabilisation (42) s'étendant radialement vers l'intérieur depuis la surface interne du boîtier (120) entre la première région d'extrémité et la seconde région d'extrémité du boîtier (120) et disposé de manière adjacente à l'élément de verrouillage (152) et espacé de celui-ci de manière circonférentielle; **caractérisé en ce que** l'accessoire comprend en outre:
un capuchon de biopsie (44) incluant un ou plusieurs éléments d'étanchéité pour assurer l'étanchéité contre le port de biopsie (10), où le capuchon de biopsie (44) est disposé à l'intérieur du boîtier (120).

12. Accessoire (118) selon la revendication 11, comprenant en outre un second élément de verrouillage (152) s'étendant depuis la surface interne du boîtier (120) et disposé à l'opposé de l'élément de verrouillage (152).

13. Accessoire (118) selon l'une quelconque des revendications 11 et 12, où l'élément de verrouillage (152) inclut une région courbée (40).

14. Accessoire (118) selon l'une quelconque des revendications 11 à 13, où l'élément de verrouillage (152) est sensiblement en forme de V.
